# EUROPÄISCHE PATENTSCHRIFT

(11) **EP 1 374 931 B1**
(45) Veröffentlichungstag und Bekanntmachung des Hinweises auf die Patenterteilung: **01.03.2006**
(21) Anmeldenummer: 03013541.2
(22) Anmeldetag: 13.06.2003
(51) Int. Cl.: A61M 5/142, F04B 43/08, A61M 39/12, F16L 31/00, F16L 33/20

(54) **Infusionspumpe**
Infusion pump
Pompe à perfusion

(30) Priorität: 21.06.2002 DE 20209663 U
(43) Veröffentlichungstag der Anmeldung: 02.01.2004
(73) Patentinhaber: B. BRAUN MELSUNGEN AG, 34212 Melsungen (DE)
(72) Erfinder: Knuth, Reinhard, 34212 Melsungen (DE); Lutz, Ronald, 52222 Stolberg (DE); Schacht, Bodo, 34323 Malsfeld (DE)
(74) Vertreter: Selting, Günther

(56) Entgegenhaltungen:
- DE-A- 4 126 087
- DE-U- 8 406 203
- DE-U- 29 720 382
- US-A- 3 565 554
- US-A- 5 215 450

## Beschreibung

Die Erfindung betrifft eine Infusionspumpe mit einem Pumpenschlauch, der an entgegengesetzten Enden je ein Übergangsstück aufweist, einem einen Pumpenfingermechanismus enthaltenden Gehäuse mit zwei Halterungen zur Befestigung der beiden Übergangsstücke und einer an dem Gehäuse vorgesehenen Tür, die ein Widerlager zum Abstützen des Pumpenschlauchs bildet.

Aus DE 8406203 U1 der Anmelderin ist eine Infusionspumpe bekannt, die ein Gehäuse zur Aufnahme eines auswechselbaren Pumpenschlauchs aufweist. In dem Gehäuse ist ein Pumpenfingermechanismus vorgesehen, der den Schlauch von oben nach unten fortlaufend zusammenquetscht und dadurch eine volumetrische Förderung der in dem Pumpenschlauch enthaltenen Flüssigkeit bewirkt. Der Pumpenschlauch besteht aus einem relativ weichflexiblem Material, insbesondere Silikon, und er ist an seinen beiden Enden mit Übergangsstücken versehen, an die ein Zuleitungsschlauch bzw. ein Ableitungsschlauch aus einem flexiblen Kunststoffmaterial angeschlossen ist. An dem Gehäuse sind Halterungen vorgesehen, in die die Übergangsstücke eingehakt werden. Auf diese Weise ist es möglich, den Pumpenschlauch in definierter Weise an dem Gehäuse zu befestigen. Die beiden Halterungen und die zugehörigen Übergangsstücke sind so ausgebildet, dass das obere Übergangsstück nur in die obere Halterung und das untere Übergangsstück nur in die untere Halterung passt. Die Länge des Pumpenschlauchs macht es aber möglich, dass der Pumpenschlauch in sich verdreht werden kann, wobei beispielsweise die untere Halterung gegenüber der oberen Halterung um 360° verdreht ist. Dadurch besteht die Gefahr einer größeren Abweichung der eingestellten Fördermenge oder schlimmstenfalls ein freier Durchfluss (Free-Flow).

Der Oberbegriff des Patentanspruchs 1 geht aus von einer Infusionspumpe, die in DE 41 26 087 A1 der Anmelderin beschrieben ist. Bei dieser Infusionspumpe sind die Halterungen, an denen die Enden des Pumpenschlauchs befestigt sind, jeweils mit Lochösen ausgebildet, wobei die Lochösen am saugseitigen Ende des Schlauchs eine andere Form und/oder Größe als die Lochösen am druckseitigen Ende des Schlauches haben. Die Lochösen sind auf angepasste Zapfen der Pumpe aufsteckbar. Diese Zapfen können schräg nach oben abstehen, wobei die Lochösen aus Bohrungen bestehen, die senkrecht in den entsprechenden Laschen der Halterungen verlaufen.

Der vorliegenden Erfindung liegt die Aufgabe zugrunde, eine Infusionspumpe mit Pumpenschlauch so zu gestalten, dass bei einem falschen Einlegen des Pumpenelements (Rückseite vorne) der Fehler sofort sichtbar ist bzw. ein Betrieb der Pumpe unmöglich ist.

Die Lösung dieser Aufgabe erfolgt erfindungsgemäß mit den im Anspruch 1 angegebenen Merkmalen. Hiernach greift mindestens eine der Halterungen mit dem zugehörigen Übergangsstück durch mindestens eine Kombination eines schrägen Zapfens und eines darauf aufschiebbaren schrägen Loches zusammen, wobei Zapfen und Loch in bezug auf die Längsachse des eingelegten Pumpenschlauchs die selbe Schräge haben.

Bei ordnungsgemäß eingelegtem Pumpenschlauch greift der schräge Zapfen passend in das Loch, das die gleiche Schräge aufweist, so dass das Übergangsstück sich flach an das Gehäuse anlegt. Bei falschem Ansetzen des Übergangsstücks an das Gehäuse steht das Übergangsstück schräg zum Gehäuse, so dass eine Aufwölbung entsteht, die das Schließen der Tür unmöglich macht. Ein ordnungsgemäßer Betrieb der Pumpe wird bei fehlerhaftem Einlegen des Schlauchs (Rückseite vorne) unmöglich gemacht. Die Erfindung schließt ein fehlerhaftes Einlegen des Pumpenschlauchs mit hoher Sicherheit aus.

Vorzugsweise ist der mindestens eine schräge Zapfen an der Halterung und das mindestens eine schräge Loch an dem Übergangsstück angeordnet. Ferner sind bei einer bevorzugten Ausführungsform der Erfindung an der Halterung zwei parallele Zapfen und an dem Übergangsstück zwei parallele Löcher vorgesehen. Es besteht aber auch die Möglichkeit, den Zapfen am Übergangsstück und das Loch an der Halterung vorzusehen.

Das Loch ist nicht nur eine bloße Öffnung in einer flachen Platte, sondern ein zylindrischer schräg zur Mittelebene des Übergangsstück verlaufender Kanal.

Gemäß einer bevorzugten Ausgestaltung der Erfindung ist vorgesehen, dass die beiden Halterungen von unterschiedlicher Bauart sind, wobei jedes der beiden Übergangsstücke der Bauart der zugehörigen Halterung angepasst ist. Auf diese Weise wird verhindert, dass bei dem Pumpenschlauch oben und unten verwechselt wird, weil das untere Übergangsstück nicht mit der oberen Halterung zusammenpasst und das obere Übergangsstück nicht mit der unteren Halterung zusammenpasst.

Vorzugsweise ist der Pumpenschlauch mit einem längslaufenden Farbstreifen versehen. Auf diese Weise können Torsionsverdrehungen des Pumpenschlauchs optisch leicht erkannt werden.

Die Erfindung befasst sich ferner mit dem Problem, an einem Pumpenschlauch die Anbringung der Übergangsstücke zu verbessern. Der weich-elastische Pumpenschlauch, der zumeist aus Silikon besteht, kann mit anderen Komponenten nicht verklebt oder verschweißt werden. Es ist eine mechanische Verbindung zwischen diesen Teilen erforderlich. Bisher wird das Ende des Pumpenschlauchs auf einen Rohrzapfen des Übergangsstücks aufgeschoben. Anschließend wird ein Spannring um das aufgeschobene Ende des Pumpenschlauchs herumgelegt, um diesen auf dem Rohrzapfen dichtend festzulegen.

Eine weitere Aufgabe der Erfindung besteht darin, einen Pumpenschlauch mit an beiden Enden vorgesehenen Übergangsstücken zu schaffen, der eine hohe Zugfestigkeit sowie eine wesentlich verbesserte Druckfestigkeit aufweist und einfach herstellbar ist.

Die Lösung dieser Aufgabe erfolgt erfindungsgemäß mit den Merkmalen des Anspruchs 7.

Im folgenden wird unter Bezugnahme auf die Zeichnungen ein Ausführungsbeispiel der Erfindung näher erläutert.

Es zeigen:
- Fig. 1: eine perspektivische Darstellung eines Teiles der Infusionspumpe mit einem Gehäuse und einem daran auswechselbar befestigten Pumpenschlauch,
- Fig. 2: einen schematischen Vertikalschnitt durch die obere Halterung und das daran befestigte Übergangsstück und
- Fig. 3: eine Frontansicht des am Gehäuse befestigten Pumpenschlauchs.

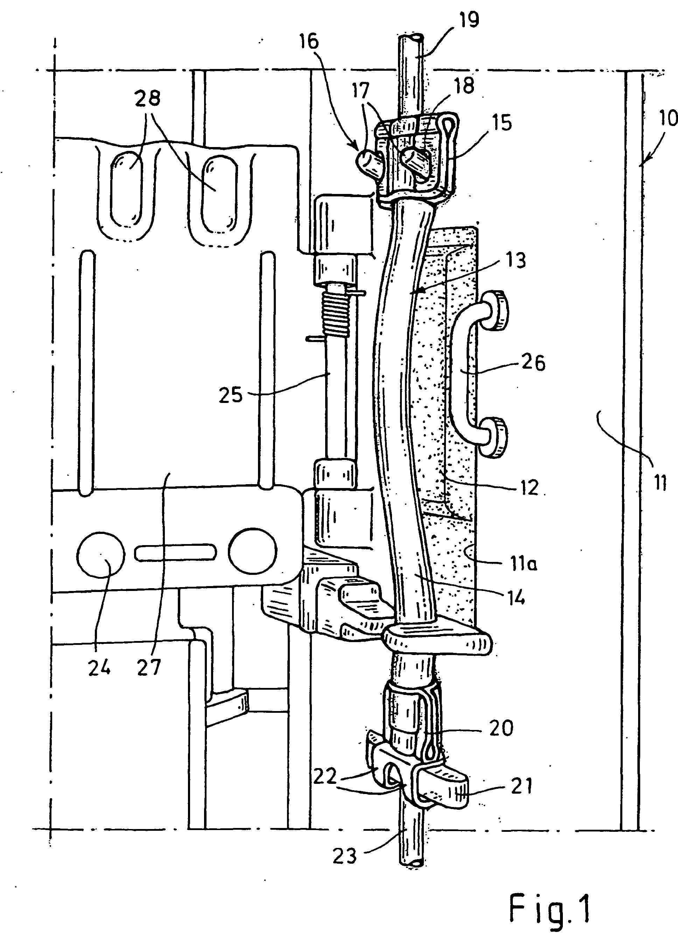

Die in Figur 1 dargestellte Infusionspumpe weist ein Gehäuse 10 auf, das generell in ähnlicher Weise ausgebildet ist wie das Gehäuse der Infusionspumpe nach DE 8406203 U1. An einer Frontwand 11 des Gehäuses 10 befindet sich eine Öffnung 11a, hinter der ein Pumpenfingermechanismus 12 einer (nicht dargestellten) Fingerpumpe angeordnet ist. Die Fingerpumpe weist zahlreiche hintereinander angeordnete Finger auf, die nacheinander von oben nach unten gegen den Pumpenschlauch gedrückt werden und diesen flachdrücken, so dass die darin enthaltene Flüssigkeit von oben nach unten peristaltisch transportiert wird. Der Pumpenfingermechanismus 12 ist mit einer elastischen Abdeckung bedeckt.

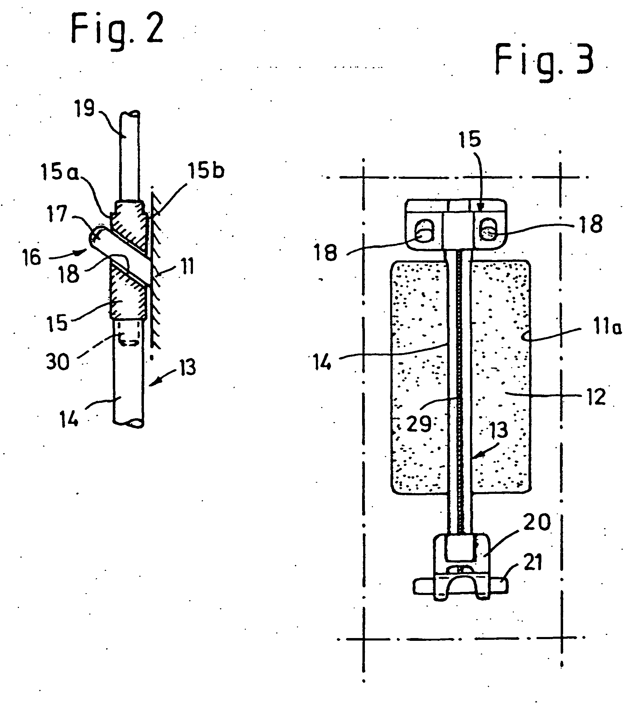

Vor der Öffnung 11a wird der Pumpenschlauch 13 installiert. Der Pumpenschlauch 13 besteht aus einem weich-elastischen Schlauchstück 14, vorzugsweise aus Silikon. Die Länge beträgt ca. 90 mm, der Innendurchmesser 4 mm und der Außendurchmesser 6 mm. An dem oberen Ende des Schlauchstücks 14 ist das obere Übergangsstück 15 befestigt. Dieses Übergangsstück ist in die obere Halterung 16 des Gehäuses 10 eingehängt. Diese Halterung 16 weist zwei nebeneinander und auf gleicher Höhe angeordnete Zapfen 17 auf, die von der Vorderwand 11 des Gehäuses schräg nach oben abstehen, wie dies in Figur 2 dargestellt ist. Das Übergangsstück 15 hat zwei schräg nach oben gerichtete kanalförmige Löcher 18, deren Achse unter dem selben Winkel verläuft wie diejenige der Zapfen 17. Das Übergangsstück 15 ist mit einem Zulaufschlauch 19 aus einem flexiblen Kunststoff verbunden.

Das Übergangsstück hat eine Vorderseite 15a und eine Rückseite 15b. Die Rückseite 15b ist der Frontwand 11 des Gehäuses zugewandt. Würde das Übergangsstück in der Weise montiert werden, dass seine Vorderseite 15a der Frontwand 11 des Gehäuses zugewandt ist, so würde sich das Übergangsstück nicht flach gegen die Frontwand 11 des Gehäuses legen, sondern schräg von diesem abstehen.

An dem unteren Ende des Pumpenschlauchs 13 befindet sich das untere Übergangsstück 20, das an einer Halterung 21 des Gehäuses eingehakt ist. Das Übergangsstück 20 weist zwei hakenförmige Rastklammern 22 auf, die die Halterung 21 übergreifen. Die Halterung 21 besteht aus einer querlaufenden Leiste, die an der Frontwand 11 angeformt ist. In der Leiste befindet sich eine Öffnung für den Durchgang des Ablaufschlauchs 23, der mit dem Übergangsstück 20 verbunden ist. Die Übergangsstücke 15,20 bilden zusammen mit dem Schlauchstück 14 den Pumpenschlauch 13. Der Pumpenschlauch 13 bildet mit dem Zulaufschlauch 19 und dem Ablaufschlauch 23 ein Schlauchset, das einen Einmalartikel darstellt, welcher nach Gebrauch erneuert wird.

Als Widerlager für den Pumpenschlauch 13 ist am Gehäuse 10 eine Tür 24 vorgesehen, die um eine vertikale Achse 25 schwenkbar ist und an einem Verschlussteil 26 verriegelt wird. Die Tür 24 ist dem Pumpenfingermechanismus 12 gegenüber angeordnet, und sie weist eine Anlagefläche 27 auf, an der das Schlauchstück 14 von dem Pumpenfingermechanismus 12 flachgedrückt wird. Oberhalb der Anlagefläche 27 befinden sich in der Tür 24 Aushöhlungen 28 zur Aufnahme der Enden der Zapfen 17 der Halterung 16.

Figur 3 zeigt eine Frontansicht des ordnungsgemäß eingesetzten Pumpenschlauchs 13. An der Vorderseite des Schlauchstücks 14, die für den Bediener sichtbar ist, wenn die Tür 24 geöffnet ist, befindet sich ein längslaufender Farbstreifen 29. Der Farbstreifen besteht aus Lebensmittelfarbe und wird bei der Extrusion des Schlauchs im Abrollverfahren vor dem Aushärten des Schlauchmaterials aufgetragen. Der Farbstreifen 29 würde erkennbar machen, wenn das untere Übergangsstück 20 um 360° verdreht wäre, so dass das Schlauchstück 14 schraubenförmig verwunden wäre.

## Patentansprüche

1. Infusionspumpe mit einem Pumpenschlauch (13), der an entgegengesetzten Enden je ein Übergangsstück (15,20) aufweist, einem einen Pumpenfingermechanismus (12) enthaltenden Gehäuse (10) mit zwei Halterungen (16,21) zur Befestigung der beiden Übergangsstücke (15,20) und einer an dem Gehäuse (10) vorgesehenen Tür (24), die ein Widerlager zum Abstützen des Pumpenschlauchs (13) bildet,
**dadurch gekennzeichnet, dass**
mindestens eine der Halterungen (16) mit dem zugehörigen Übergangsstück (15) durch mindestens eine Kombination eines schrägen Zapfens (17) und eines darauf aufschiebbaren schrägen Loches (18) zusammengreift, wobei der Zapfen (17) und das Loch (18) in bezug auf die Längsachse des eingelegten Pumpenschlauches (13) die selbe Schräge haben.

2. Infusionspumpe nach Anspruch 1, **dadurch gekennzeichnet, dass** der schräge Zapfen (17) an der Halterung (16) und das schräge Loch (18) an dem Übergangsstück (15) angeordnet ist.

3. Infusionspumpe nach Anspruch 1 oder 2, **dadurch gekennzeichnet, dass** die Halterung (16) zwei parallele schräge Zapfen (17) und das Übergangsstück (15) zwei parallele Löcher (18) aufweist.

4. Infusionspumpe nach einem der Ansprüche 1 - 3, **dadurch gekennzeichnet, dass** in der Tür (24) Aushöhlungen (28) zur Aufnahme der Enden der Zapfen (17) vorgesehen sind.

5. Infusionspumpe nach einem der Ansprüche 1 - 4, **dadurch gekennzeichnet, dass** das andere Übergangsstück (20) eine einen Steg des Gehäuses (10) übergreifende Rastklammer (22) aufweist.

6. Infusionspumpe nach einem der Ansprüche 1 - 5, **dadurch gekennzeichnet, dass** der Pumpenschlauch (13) einen längslaufenden Farbstreifen (29) zur Erkennung von Schlauchverdrehungen aufweist.

## Claims

1. An infusion pump with a pump hose (13) with a respective transition piece (15, 20) at opposite ends, a housing (10) accommodating a pump finger mechanism (12) and having two holders (16, 21) for fastening the two transition pieces (15, 20), and a door (24) provided at the housing (10) and forming a counter bearing for supporting the pump hose (13),
**characterized in that**
at least one of the holders (16) mates with the associated transition piece (15) via at least a combination of an oblique pin (17) and an oblique hole (18) to be slipped thereon, the pin (17) and the hole (18) being inclined the same with respect to the longitudinal axis of the placed pump hose (13).

2. The infusion pump of claim 1, wherein the oblique pin (17) is provided at the holder (16) and the oblique hole (18) is provided at the transition piece (15).

3. The infusion pump of claim 1 or 2, wherein the holder (16) has two parallel oblique pins (17) and the transition piece (15) has two parallel holes (18).

4. The infusion pump of claim 1, wherein the door (24) is provided with recesses (28) for receiving the ends of the pins (17).

5. The infusion pump of one of claims 1 - 3, wherein the other transition piece (20) comprises a locking clamp (22) engaging over a web of the housing (10).

6. The infusion pump of one of claims 1-5, wherein the pump hose (13) has a longitudinal color strip (29) for detecting hose twisting.

## Revendications

1. Pompe à perfusion comprenant un tuyau pour pompe (13) présentant, à des extrémités opposées, respectivement un adaptateur (15, 20), un boîtier (10) contenant un mécanisme formant pompe à tuyau flexible (12), avec deux supports (16, 21) destinés à la fixation des deux adaptateurs (15, 20), et une porte (24) prévue sur le boîtier (10), qui constitue un contrefort sur lequel s'appuie le tuyau pour pompe (13),
**caractérisée en ce qu'**
au moins l'un des supports (16) coopère avec l'adaptateur (15) qui lui est associé, par au moins une combinaison d'un doigt oblique (17) et d'un trou oblique (18) susceptible d'être enfilé sur lui, le doigt (17) et le trou (18) présentant la même oblique, par rapport à l'axe longitudinal du tuyau pour pompe (13) qui y est inséré.

2. Pompe à perfusion selon la revendication 1, **caractérisée en ce que** le doigt oblique (17) est prévu sur le support (16) et le trou oblique (18) est prévu sur l'adaptateur (15).

3. Pompe à perfusion selon la revendication 1 ou 2, **caractérisée en ce que** le support (16) présente deux doigts obliques (17) parallèles et l'adaptateur (15) présente deux trous obliques (18) parallèles.

4. Pompe à perfusion selon l'une des revendications 1 à 3, **caractérisée en ce que** sont prévus, dans la porte (24), des cavités (28) destinées à loger les extrémités des doigts (17).

5. Pompe à perfusion selon l'une des revendications 1 à 4, **caractérisée en ce que** l'autre adaptateur (20) présente une bride d'enclenchement (22) qui coiffe une nervure du boîtier (10).

6. Pompe à perfusion selon l'une des revendications 1 à 5, **caractérisée en ce que** le tuyau pour pompe (13) présente une bande de couleur (29) longitudinale permettant d'identifier une torsion du tuyau.
